# EUROPEAN PATENT APPLICATION

(11) **EP 1 029 843 A1**
(43) Date of publication of application: **23.08.2000**
(21) Application number: 00200534.6
(22) Date of filing: 16.02.2000
(51) Int. Cl.: C07C 45/72, C07C 45/74, C07C 49/24, C07C 49/203, C07C 403/16, B01J 21/16

(54) **Method for the condensation of an aldehyde and a ketone**

(30) Priority: 16.02.1999 EP 99200450
(71) Applicant: Universiteit Utrecht, 3584 CS Utrecht (NL)
(72) Inventor: Van Dillen, Adrianus Jacobus, 4102 AB Culemborg (NL); Roelofs, Jules Caspar Albert Anton, 3584 HH Utrecht (NL); Geus, John Wilhelm, 3723 GJ Bilthoven (NL); De Jong, Krijn Pieter, 3992 XA Houten (NL); Jastrzebski, Johann Thöns Barthold Heinrich, 3731 XA De Bilt (NL)
(74) Representative: Ottevangers, Sietse Ulbe

(57) **Abstract**

The invention is directed to a process for the condensation of an unsaturated aldehyde and a ketone in liquid phase, at a temperature below 60°C in the presence of anionic clay, in which at least part of the charge compensating anions has been replaced by OH⁻ions, wherein the aldehyde is R₁R₂R₃X-C(=O)H, wherein X is selected from C, Si, Ge, Sn, Pb, N, P, As, Sb wherein R₁, R₂, and R₃ are identical or different and selected from H or optionally substituted (cyclo)alkyl, (cyclo)alkenyl, alkynyl or (hereto)aromates, with the proviso that at least one on the groups R₁, R₂, and R₃ contains a non-aromatic, unsaturated carbon-carbon bond, and that in case X is three-valent, R₃ is absent.

## Description

The present invention is directed to a method for the condensation of an aldehyde and a ketone in the presence of a basic or alkaline catalyst. This type of reaction is also called the aldol condensation.

The aldol condensation is of prime importance for fine chemicals synthesis and is generally catalysed by acids or bases. In the present invention the emphasis is on the use of base catalysed aldol condensations. Most work that is reported on this type of aldol condensations was performed at relatively high temperatures, in the gas phase. Further, the work was generally done on condensation of ketones, like condensation of acetone to yield diacetone alcohol. Further a lot of work was done on the condensation of benzaldehyde with a ketone, such as acetone.

As catalyst, a dissolved alkaline compound can be used, although some work has also been done on the reaction of benzaldehyde and substituted benzaldehydes with acetone, catalysed by Mg-Al hydrotalcites.

As indicated above, aldol condensations can be used for the production of a variety of fine chemicals. An important group of reactants for aldol condensation compounds, contains the group of organic compounds wherein the aldehyde function is not linked to an aromatic carbon. An important example of this group of compounds is citral, which can be reacted with acetone to yield pseudoionone. Pseudoionone and derivatives thereof are important intermediate products in the development of said synthetic perfumery chemicals.

The added condensation of aldehydes with ketones is for instance disclosed in an article of Noda et al. (C. Noda et al.; Brazilian Journal of Chemical Engineering 1998, vol.15, pp 120-125). This article describes the condensation of citral with acetone at a temperature of 57°C using basic solid catalysts such as calcined hydrotalcite, which means that the original hydrotalcite structure is converted into a mixture of the constituent oxides. Conversion was reported as not significant. Only at elevated temperatures (398K) and increased pressure and with a calcined hydrotalcite catalyst an acceptable conversion could be obtained. It was suggested that at further increased temperatures improvements could be found.

Another important aspect of the aldol condensation is the type of catalyst to be used. Although generally homogeneous catalysts have found application, with the corresponding difficulty in recovery thereof, the use of solid base catalysts has attracted more attention recently.

Surprisingly it has now been found that unsaturated aldehydes are suitable substrates in the aldol condensation (at only slightly elevated temperatures) when an anionic clay is used in which at least part of the charge composition carbonate ions have been replaced by OH⁻ ions.

The present invention is directed to a method for the condensation of an unsaturated aldehyde and a ketone in liquid phase, at a temperature below 60°C in the presence of anionic clay, in which at least part of the charge compensating anions has been replaced by OH^{-―} ions, wherein the aldehyde is R₁R₂R₃X-C(=O)H, wherein X is selected from C, Si, Ge, Sn, Pb, N, P, As, Sb wehrein R₁, R₂, and R₃ are identical or different and selected from H or optionally substituted (cyclo)alkyl, (cyclo)alkenyl, alkynyl or (hereto)aromates, with the proviso that at least one on the groups R₁, R₂, and R₃ contains a non-aromatic, unsaturated carbon-carbon bond, and that in case X is three-valent, R₃ is absent.

In accordance with the invention a reaction is carried out in liquid phase, as opposed to the conventional gas phase reactions, preferably at a temperature below 60°C, preferably at atmospheric pressure. As catalyst an anionic clay material is used, preferably a hydrotalcite type clay. More in particular, the clay may be modified with bulky anions or neutral molecules, thereby providing an improved catalyst having superior selectivity and activity.

The clay material to be used in accordance with the present invention is an anionic clay, of the hydrotalcite type, consisting of layered clay material, wherein part of the cations in the layers is exchanged and a charge deficit is obtained in the layers, which is compensated by anions between the layers.

The hydrotalcite anionic clay to be used as a catalyst can be a natural or synthetic hydrotalcite clay, optionally modified with various anions or neutral molecules.

As indicated above, this type of clay has a layered structure, consisting of cations octahedrally surrounded with hydroxyl groups. By using a combination of cations of different valencies, a combination of three valent cations with one-, two- or four-valent cations a charge deficit is created between the layers of the octahedrally surrounded cations. This charge deficit is usually compensated by the presence of carbonate-ions between the layers. The presence of the anions between the layers provides the catalytic properties of the material.

It is, however, preferred to modify this layered structure by exchanging the carbonate between the layers for other anions, thereby modifying the structure of the clay and/or the anionic properties.

When these anions are of a different size than carbonate, these anions force the positively charged layers from each other thereby modifying the clay with the result that reactants may enter the clay material and may react with each other in the presence of the negatively charged anions between the layers. By carefully selecting the anions, such as on size or charge, it is possible to tailor the catalyst for a specific reaction, for example on size specific features or on ion strength or base strength. Examples of suitable anions are selected from the group of bicarboxylic acids, such as oxalate, malonate, succinate adipate, sebasate and/or from the group of polyanions such as Mo₇O₂₄⁶⁻, B(OH)₄⁻ [B₃O₃(OH)₄]⁻, B₃O₃(OH)₅]²⁻ B₄O₅(OH)₄]²⁻, V₄O₁₂⁴⁻, HV₂O₇³⁻, V₃O₉³⁻ and/or anions derived from alcohols such as methoxide, ethoxide or tert-butoxide or phenoxide and the like or anions derived from polyalcohols such as glycol or glycerol and the like and/or anions derived from other compounds with free hydroxyl groups such as ethylene glycol or polyethylene glycols and the like. Especially OH⁻ is a suitable anion. Alternative anions that can be used are halogenides, NO₃⁻ and NO₂⁻. Examples of suitable neutral molecules that can be used are ethylene glycol and glycerol.

The aldol condensation according to the invention is done with an aldehyde and a ketone, whereby the aldehyde is defined in accordance with the formula given above. An important aspect of the aldehydes to be used in the invention is the non-aromatic character of the compounds, that is to say that the aldehyde group is not bound to a carbon atom being part of an aromatic ring. According to the invention it has been found that by having the aldehyde not bound to an aromatic carbon atom various process advantages may be obtained, especially in terms of selectivity and reaction rate.

The aldehydes to be used in accordance with the invention have the aldehyde function bound to a linking atom, which may either be an aliphatic carbon atom or another atom from the group of Si, Ge, Sn, Pb, N, P, As and Sb. Connected to this linking atom are two or three organic moieties (the number depending on the valency of the linking atom). The organic moieties connected to the linking atom may be the same or different and are selected from H or optionally substituted (cyclo)alkyl, (cyclo)alkenyl, alkynyl or (hetero)aromates. It is to be noted that the organic moieties may contain aromatic or other non-aliphatic substituents or components, such as silicons or halogens.

As indicated above one of the important compounds to be used as aldehyde in the process of the present invention is citral. By reacting citral with acetone or 2-butanone the reaction products are pseudoionones, which are important intermediates in the preparation of vitamin A and various aroma chemicals.

The other reactant in this system is a ketone, generally a ketone with up to about six carbon atoms. Suitable ketones are for example acetone and methyl ethyl ketone.

Optimal reaction conditions for the condensation reaction between the aldehyde and the ketone are relative easy to determine for the skilled person. An important aspect of the invention is to keep the system in the liquid phase at low temperature. In order to have full benefit of the advantageous properties of the catalytic system, these conditions are important. Other aspects that need to be taken into account when determining the process conditions are the amount of catalyst in the system, which may range from 1 to 50 wt.%. In a preferred embodiment, wherein the aldehyde and the catalyst are employed in a ratio in the range of 0.1-25 mmol aldehyde/g catalyst, preferably 0.5-10, more preferably 0.7-7. Preferably the amount of citral to catalyst does not exceed 100 mmol/g catalyst. The actual amount is further determined by the reactants, the actual structure of the catalyst and any other reaction conditions that may influence it. The actual operation of the process is further influenced by the fact that the ketone has the property to self condensate, which means that the concentration of the ketone should preferably be kept low, for example by slowly adding it during the reaction. Self-condensation is even more important in the case of aldehydes. Accordingly the same procedure may be used for the addition of aldehydes.

The catalyst can be prepared from natural hydrotalcite, by calcination to drive off water and carbon dioxide. By rehydrating the material subsequently, under exclusion of carbon dioxide or carbonate, in the presence of the required anions, the clay is restructured again to layers, with the anions in between the layers.

It is also possible to produce synthetic hydrotalcite like anionic clay materials and to use these in the reactors according to the invention.

### Synthesis modified hydrotalcite-like material

Synthesizing the modified hydrotalcite-like catalyst comprises adding a solution containing one or more divalent metal ions selected from the group consisting of Mg²⁺, Zn²⁺, Cu²⁺, Ni²⁺, Co²⁺, Mn²⁺ and Fe²⁺ and one or more trivalent metal ions selected from the group consisting of Al³⁺, Fe³⁺, Co³⁺, Mn³⁺ and Cr³⁺ to a solution with a pH chosen between 6 and 14. (containing anions of the group consisting of carbonate, nitrate, hydroxide, chloride, sulphate and others) forming a mixture, and thereafter recovering a material of the formula:

M²⁺₂ₓM³⁺₂(OH)₄ₓ₊₄Aⁿ⁻¹ _{2/n} ·Z H₂O,

wherein x varies between 1.5 and 5, n is 1, 2, 3 or 4, A is an anion, depending on the nature of the used solution, Z varies between 0 and 5. After drying, the hydrotalcite-like material is modified which comprises calcining the hydrotalcite-like material with a rate of 1°C/min to 400-700°C, preferably 450°C, maintaining this temperature for 6-16 hours, preferably 8 hours, cooling the calcined hydrotalcite-like material with a rate of 1-10°C/min, preferably 5°C/min, in an inert atmosphere, preferably nitrogen or helium, to 15-40°C, preferably 30°C, rehydrating the calcined hydrotalcite-like material in an inert gas flow of nitrogen or helium, containing 1-15% water during 6-24 hours, preferably 18-20 hours until the 003/001 reflection in XRD is restored. The rehydrated material consists of the formula:

M²⁺₂ₓM³⁺₂(OH)₄ₓ₊₆ Aⁿ⁻¹ _{2/n} ·Z H₂O,

wherein x varies between 1.5 and 5, n is 1, 2, 3 or 4 and Z varies between 0 and 5. This hydrotalcite-like material can further be modified by replacing (part of) the OH⁻ anions by anions selected from the group consisting of bicarboxylic acids such as oxalate, malonate, succinate, adipate, sebasate, polyanions such as Mo₇O₂₄⁶⁻, B(OH)₄⁻, [B₃O₃(OH)₄]⁻, B₃O₃(OH₅)]²⁻, B₄O₅(OH)₄]²⁻, V₄O₁₂⁴⁻, HV₂O₇³⁻, V₃O₉³⁻, V₁₀O₂₈⁶⁻, glycerol, ethylene glycol, alkoxides, halogenides and NO₃⁻, NO₂⁻ and OH⁻, preferably OH⁻. This hydrotalcite-like material can also be modified by the inclusion of neutral molecules such as ethylene glycol or glycerol.

### Example synthesis modified hydrotalcite-like material:

A 500 ml, 3-necked round bottomed flask was equipped with a reflux condenser. This flask was heated using a water bath and a IKAMAG RET digi-visc applied with a Pt 100 heating sensor. To this 3-neck flask was brought a solution containing 60 g water, 14 g NaOH and 10 g Na₂CO₃. A solution containing 25.6 g Mg(NO₃)₂·6H₂O, 18.7 g Al(NO₃)₃·9H₂O and 55 g water was added. The slurry was stirred at 1100 rpm at 60°C for a period of 24 hours. After this, the slurry was filtrated and washed with 3x250 ml water. The residue was dried overnight at 100°C and characterised as hydrotalcite by its XRD pattern. ICP-analysis revealed a Mg/Al ratio of 2.0. The BET surface area for this material was 88 m²/g. After crushing, the solid was heated in air with a rate of 10°C/min up to 450°C in a Thermolyne 79300 tube furnace, kept at this temperature for 8 hours and cooled down to 30°C. The product was characterised to be a mixture of MgO and Al₂O₃, having a BET surface area of 253 m²/g. The solid was rehydrated in a flow of nitrogen containing 10% (vol) of water until the hydrotalcite reflections in the XRD pattern had reappeared. The BET surface area of the rehydrated product was 57 m²/g.

### Examples condensation reaction:

### Example 1

0.05 mole citral, 2 mole acetone and 0.3 g rehydrated hydrotalcite (0.5 wt.%) were stirred at 800 rpm in a 200 ml cooled glass reactor equipped with baffles. During the first 4 hours, aliquots were taken every 10 minutes and the catalyst was removed by filtration. After 23 hours, 66% citral was converted with a selectivity of 90% to the corresponding aldol condensation products, pseudoionone and the corresponding β-hydroxy keton analogue of pseudoionone.

### Example 2

0.05 mole citral, 2 mole acetone and 2.0 g rehydrated hydrotalcite (2 wt%) were stirred at 800 rpm in a 500 ml round bottomed flask equipped with a reflux condenser with a IKAMAG RET digi-visc applied with a Pt 100 heating sensor, at room temperature for 5 hours. Aliquots were taken every 30 minutes and the catalyst was removed by centrifuging the aliquots at 3000 rpm for 3 minutes with a Centauer 2 MSE centrifuge. GC/MS analysis pointed out the presence of aldol condensation products pseudoionone and the corresponding β-hydroxy keton analogue of pseudoionone.

### Example 3

0.05 mole citral, 140 ml acetone/MeOH (1:1) and 2.0 g rehydrated hydrotalcite (2 wt%) were stirred at 800 rpm in a 500 ml round bottomed flask equipped with a reflux condenser with a IKAMAG RET digi-visc applied with a Pt 100 heating sensor, at room temperature for 5 hours. Aliquots were taken every 30 minutes and the catalyst was removed by centrifuging the aliquots at 3000 rpm for 3 minutes with a Centauer 2 MSE centrifuge. GC/MS analysis pointed out the presence of aldol condensation products pseudoionone and the corresponding β-hydroxy keton analogue of pseudoionone.

## Claims

1. Method for the condensation of an unsaturated aldehyde and a ketone in liquid phase, at a temperature below 60°C in the presence of anionic clay, in which at least part of the charge compensating anions has been replaced by OH⁻ions, wherein the aldehyde is R₁R₂R₃X-C(=O)H, wherein X is selected from C, Si, Ge, Sn, Pb, N, P, As, Sb wherein R₁, R₂, and R₃ are identical or different and selected from H or optionally substituted (cyclo)alkyl, (cyclo)alkenyl, alkynyl or (hereto)aromates, with the proviso that at least one on the groups R₁, R₂, and R₃ contains a non-aromatic, unsaturated carbon-carbon bond, and that in case X is three-valent, R₃ is absent.

2. Method according to claim 1 wherein the basic layered clay is of the hydrotalcite type.

3. Method according to claim 1 or 2 wherein the basic layered clay is modified with anions or neutral molecules.

4. Method according to any of the preceding claims wherein the anions are selected from the group of bicarboxylic acids, such as oxalate, malonate, succinate adipate, sebasate and/or from the group of polyanions such as Mo₇O₂₄⁶⁻, B(OH)₄⁻ [B₃O₃(OH)₄]⁻, B₃O₃(OH)₅]²⁻ B₄O₅(OH)₄]²⁻, V₄O₁₂⁴⁻, HV₂O₇³⁻, V₃O₉³⁻ and/or anions derived from alcohols such as methoxide, ethoxide or tert-butoxide or phenoxide and the like or polyalcohols such as glycol or glycerol and the like and/or other compounds with free hydroxyl groups such as ethylene glycol or polyethylene glycols and the like and/or halogenides, NO₃⁻ and NO₂⁻, preferably OH⁻.

5. Method according to claim 4, wherein the anion is a hydroxyl ion.

6. Method according to any of the preceding claims wherein the anionic clay is modified with cations.

7. Method according to any of the preceding claims wherein the cations are selected from the group of Zn, Cu, Ni, Co, Mn, Cr, Fe, Mg, Al, Zr and Li.

8. Method according to any of the preceding claims wherein the aldehyde is citral.

9. Method according to any of the preceding claims wherein the ketone is selected from the group of non-aromatic ketones, preferably acetone or 2-butanone.

10. Method for the preparation of an optionally substituted pseudoionone or a ionone which comprises the condensation of an aldehyde with a ketone in the presence of a hydrotalcite.

11. Method according to claim 9 wherein aldehyde is citral.

12. Method according to claim 9 or 10 wherein the ketone is acetone or 2-butanone.

13. Anionic clay with charge compensating anions, wherein the compensating anions have been selected from the group of bicarboxylic acids, such as oxalate, malonate, succinate adipate, sebasate and/or from the group of polyanions such as Mo₇O₂₄⁶⁻, B(OH)₄⁻ [B₃O₃(OH)₄]⁻, B₃O₃(OH)₅]²⁻ B₄O₅(OH)₄]²⁻, V₄O₁₂⁴⁻, HV₂O₇³⁻, V₃O₉³⁻ and/or from alcohols such as methoxide, ethoxide or tert-butoxide or phenoxide and the like and/or derived from polyalcohols such as glycol or glycerol and the like and/or derived from other compounds with free hydroxyl groups such as ethylene glycol or polyethylene glycols and the like and/or halogenides, NO₃⁻ and NO₂⁻, preferably OH⁻.

14. Clay according to claim 12, for use as a catalyst in the condensation of citral with a ketone selected from acetone or 2-butanone.

15. Method for the preparation of β-hydroxyketones of the formula R₁RR₃X-C(OH)-C(=O)R₄R₅, comprising the steps of contacting an aldehyde of the formula R₁RR₃X-C(=O)H with a ketone from the formula R4C(=O)R5 with an anionic clay, wherein X is selected from C, Si, Ge, Sn, Pb, N, P, As, Sb, wherein R1, R2, and R3 are identical or different and selected from H or optionally substituted (cyclo)alkyl, (cyclo)alkenyl, alkynyl or (hetero)aromates and wherein R4 and R5 are selected from optionally substituted (cyclo)alkyl, (cyclo)alkenyl, alkynyl or (hetero)aromates.

16. Pseudoionone or ionone obtainable by the condensation of citral with acetone or 2-butanone in the presence of an anionic clay.

17. Method according to claims 1-9 wherein the aldehyde and the catalyst are employed in a ratio in the range of 0.1-25 mmol aldehyde/g catalyst, preferably 0.5-10, most preferably 0.7-7.
